# EUROPÄISCHE PATENTSCHRIFT

(11) **EP 1 970 042 B1**
(45) Veröffentlichungstag und Bekanntmachung des Hinweises auf die Patenterteilung: **02.03.2011**
(21) Anmeldenummer: 07113977.8
(22) Anmeldetag: 08.08.2007
(51) Int. Cl.: A61K 6/00, A61K 6/083

(54) **Verwendung von polymerisierbaren Calix[n]arenen als Dentalmaterialien**
Use of polymerisable Calix[n]arenes as dental materials
Utilisation de Calix[n]arènes polymérisables comme matériaux dentaires

(30) Priorität: 29.08.2006 DE 102006040439; 30.07.2007 DE 102007035734
(43) Veröffentlichungstag der Anmeldung: 17.09.2008
(73) Patentinhaber: Ivoclar Vivadent AG, 9494 Schaan (LI); Heraeus Kulzer GmbH, 63450 Hanau (DE)
(72) Erfinder: Moszner, Norbert, 9495, Triesen (LI); Ritter, Helmut, 42111, Wuppertal (DE); Fischer, Urs-Karl, 9320, Arbon (CH); Tabatabai, Monir, 40225, Düsseldorf (DE); Schaub, Matthias, 63589, Linsengericht (DE); Utterodt, Andreas, 61267, Neu-Anspach (DE)
(74) Vertreter: Fitzner, Uwe

(56) Entgegenhaltungen:
- WO-A-2005/056741
- JP-A- 9 263 560
- US-A- 4 699 966

## Beschreibung

Die vorliegende Erfindung betrifft die Verwendung von polymerisierbaren Calix[n]arenen für Dentalmaterialien mit geringem Polymerisationsschrumpf und vergleichbaren mechanischen Eigenschaften.

Calix[n]arene sind cyclische Oligomere, sog. 1ₙ-Metacyclophane (Schema 1, Formel **1**), die durch Kondensation von p-Alkylphenolen mit Formaldehyd (Schema 1, Formel **2**) bzw. von Resorcin mit Aldehyden (Schema 1, Formel **3**) zugänglich sind, wobei die phenolischen OH-Gruppen in endo- (intraanular) bzw. exo-Position (extraanular) angeordnet sind. Dabei hat die besondere schüssel- oder kelchartige Konformation der Calix[n]arene zu zahlreichen Anwendungen in der Katalyse, Chromatographie, Analytik oder Sensortechnik geführt (V. Böhmer, Angew. Chem. 107 (1995) 785-818).

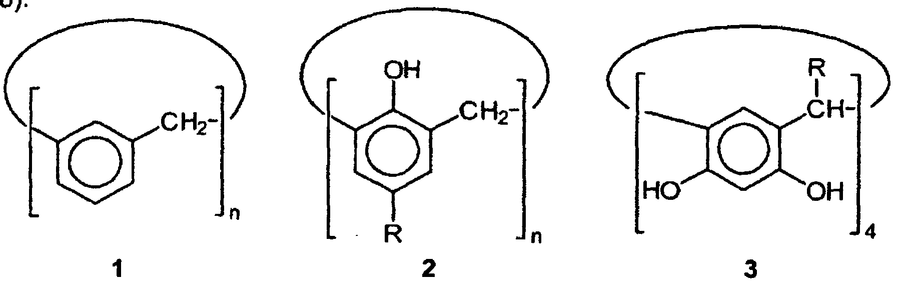

Aus der wissenschaftlichen Literatur sind eine ganze Anzahl von Arbeiten zur Synthese und Polymerisation von polymerisationsfähigen Calix[n]arenen bekannt. Beispiel hierfür sind die Synthese des radikalisch polymerisierbaren 5-[3-(Methacryloyloxy(propyl]-25,26,27,28-tetrabutoxycalix[4]arens (D. M. Gravett, J. E. Guillet (Macromolecules, 29 (1996) 617-724) bzw. des 5-(1-(Acryloyloxypropyloxymethyl)-25,26,27,28-tetra(2-ethoxyethyl)calix[4]arens (M. T. Blanda, E. Adou, Polymer 39 (1998) 3821-3826), die Synthese und radikalische Polymerisation von p-Alkylcalix[6]arenen mit (Meth)acrylgruppen, wie z.B. die Hexa(meth)acrylate von p-Methyl- oder p*-tert*-Butylcalix[6]aren, (M. lyo, K. Tsutsui, A. Kameyama, T. Nishikubo, J. Polym. Sci., Part A: Polym. Chem. 37 (1999) 3071-3078) oder die Synthese von kationisch polymerisierbaren Calix[n]arenen, wie z.B. von 5,11,17,23,29,35-hexamethyl-37,38,39,40,41,42-hexakis(allyloxy)calix[6]aren (T. Nishikubo, A. Kameyama, K. Tsutsui, M. lyo, J. Polym. Sci., Part A: Polym. Chem. 37 (1999) 1805-1814).
Die Verwendung von Calix[n]arenen in Kombination mit polymerisierbaren Formulierungen ist aus der Patentliteratur bekannt. So beschreiben z.B. US 4,636,539, US 4,718,966, US 4,912,183, EP 235,935 und FR 2,795,077 die Verwendung von nichtreaktiv modifizierten Calix[n]arenen als Beschleuniger für Cyanacrylat-Klebstoffe.
Die US 4,699,966 beschreibt mit Acrylat- oder Methacrylatgruppen funktionalisierte Calix[n]arene und Polymerisate daraus als Sequestriermittel für Metallionen. Weitere speziellere polymerisierbare Calix[n]aren- und Oxacalixarenderivate mit mindestens einer phenolischen Seitengruppe werden in US 5,216,185 beschrieben. JP 09-263560 beschreibt Calix[n]aren-Derivate die mit (Meth)acrylat, Vinyl- oder Propenylgruppen funktionalisiert sind und thermisch oder photochemisch polymerisiert werden können. JP 11-043524 beschreibt ähnliche Systeme, die jedoch zusätzlich mit Polyalkylenoxidgruppen modifiziert sind.
Die WO 2005/075398 A1 und JP 2002-088007 beschreiben polymerisierbare Calix[n]arenderivate, die in härtbaren Photoresists zu einer verbesserten Hitzebeständigkeit führen. JP 2004-137395 beansprucht einen Celluloseacrylatfilm enthaltend ein polymerisierbares Calix[n]aren-Derivat mit dem Vorteil verbesserter mechanischer und optischer Eigenschaften.
Aus der JP 2002-003563 und JP 09-263560 sind Calix[n]aren-Derivate bekannt, die polymerisierbare Gruppen (Acrylat, Methacrylat, Vinyl, Vinylether, usw.) und Säure- bzw. Anhydrid-Gruppen enthalten. Diese finden Anwendung als ätzende Resiste, Adhäsive, Lacke bzw. Beschichtungen.
Die GB 2,185,261 beschreibt eine radikalisch polymerisierbare Zusammensetzung als adhäsives Füllmaterial enthaltend ein Calix[n]arenderivat. JP 02-124850 beschreibt die Herstellung von Calix[n]arenderivaten durch Erhitzen von p-*tert*-Butyl-calix[6]arenen mit Glycidylmethacrylat und Tri-n-butylamin. US 4,617,336 und CA 1273954 beschreiben Calixarene mit Acrylatgruppen zur Stabilisierung von organischen Materialien, insbesondere Polymeren.
In JP 2000-256362 werden polymerisierbare Calix[n]arenderivate beschrieben, die mit Spiroorthoestergruppen funktionalisiert sind. Diese Verbindungen, die sich durch eine Polymerisation ohne Volumenschrumpf auszeichnen, sind u.a. zur Verwendung als Beschichtungsmaterialien geeignet.
JP 2000-264953 beschreibt schließlich härtbare Epoxy-Harze, die durch Zusatz von Calix[n]arenen vorteilhafte Eigenschaften erhalten, wie eine hohe Vernetzungsdichte, hohe Wärmebeständigkeit und gute mechanische Eigenschaften.
Die WO 2005/120229 beschreibt Stoffe, die Terpene und/oder Duftalkohole freisetzen. Die dargestellten Stoffe und Zubereitungen können unter anderem auch Calix[n]arene enthalten und dienen in erster Linie der Vermeidung der Anhaftung von Mikroorganismen an Oberflächen. Beispielsweise werden Zubereitungen beschrieben, die in Fugenmassen die Bildung von Mikroorganismen vermeiden. Ebenso wird die Verwendung von Calix[n]arenen enthaltenden Zubereitungen für die Reinigung von Prothesen beschrieben.

In der US 4,699,966 werden Calix[n]arene und aus diesen hergestellte Polymerisate beschrieben, wobei die Calix[n]arene mit einem Initiator gemischt und mittels Licht polymerisiert werden. Die Verwendung als gefülltes Dentalmaterial oder als Komponente in solchen Materialien wird nicht beschrieben.

In der US 6,117,944 A wird die Herstellung die Ermittlung der Reaktivität von verschiedenen calixarenhaltigen ungefüllten Zusammensetzungen beschrieben. Die Verwendung in Dentalmaterialien ist nicht Gegenstand dieser Patentanmeldung.

Aus der EP 1 712 537 A1 ist eine Vielzahl von verschiedenen Calix[n]aren in unterschiedlichen Zusammensetzungen bekannt. Diese enthalten allerdings keinen Füllstoff und keine weiteren radikalisch polymerisierbaren Komponenten.

Aus der EP 432990 A2 sind ungefüllte Zusammensetzungen für die Maskierung und Beschichtung von Metallen bekannt. Die Materialien werden mittels aktinischer Strahlung aus einer Lösung gehärtet. Füllstoffhaltige Dentalmaterialien mit Calix[n]aren werden nicht offenbart.

Aus der EP 196895 B1 sind ungefüllte Adhäsivzusammensetzungen bekannt, die keinen Füllstoff enthalten. Die entstehenden Polymere enthalten Calix[n]aren-Sequenzen.

Aus der WO 2005/056741 A1 sind antiadhäsive Zusammensetzungen bekannt. Diese sind ungefüllt und haben als Beschichtung auch für Prothesen und Zahn- oder Mundpflegemittel keinen direkten Bezug zu Dentalmaterialien, bei denen es um Materialien zur Erhaltung oder Wiederherstellung des Kauappartes geht. Gefüllte Zusammensetzungen für dentale Anwendungen werden nicht offenbart.

In der WO 94/15907 A1 werden Cyangruppen enthaltende Calix[n]arene beschrieben, die ungefüllt eingesetzt werden und bei denen ein geringer Schrumpf beobachtet wurde. Gefüllte Dentalmaterialien werden nicht offenbart.

Der Erfindung liegt nun die Aufgabe zugrunde, Dentalmaterialien bereitzustellen, die sich im Vergleich zu den konventionell auf üblichen Methacrylaten basierenden Materialien durch einen geringeren Polymerisationschrumpf und gute mechanische Eigenschaften auszeichnen und darüber hinaus die Realisierung von weiteren vorteilhaften Eigenschaften wie z.B. Selbsthaftung gestatten.

Diese Aufgabe wird durch die Verwendung von Zusammensetzungen gelöst, die folgende Komponenten aufweisen
(a) 0,5 bis 90 Gew.-%, besonders bevorzugt 0,5 bis 40 Gew.-% mindestens eines polymerisierbaren Calix[n]arens gemäß Formel (1),
(b) 0,01 bis 5 Gew.-%, besonders bevorzugt 0,01 bis 2 Gew.-% Initiator,
(c) 0 bis 90 Gew.-%, besonders bevorzugt 1 bis 70 Gew.-% mindestens eines weiteren kationisch und/oder radikalisch polymerisierbaren Monomers und/oder mindestens eines weiteren ringöffnend polymerisierbaren Monomers, wobei ringöffnend polymerisierbare oder mehrfach funktionelle (Meth)acrylate bevorzugt sind,
(d) 0 bis 85 Gew.-% besonders bevorzugt 3 bis 80 Gew.-% Füllstoff und
(e) 0,01 bis 5 Gew.-%, besonders bevorzugt 0,01 bis 3 Gew.-% Additiv,
wobei sich die Prozentangaben jeweils auf 100 % addieren.

Erfindungsgemäß werden vorzugsweise Calix[n]arene der allgemeinen Formel (I) eingesetzt: 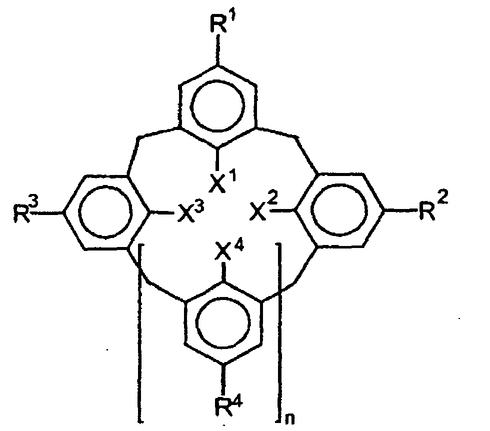
- n: = 1 bis 5
- R¹-R⁴: = unabhängig voneinander H, ein C₁- bis C₁₅-Alkylrest, der durch O unterbrochen sein kann, ein Phenyl-, oder Benzyl-Rest,
- X¹: eine Gruppe der Struktur: Y¹-Rₐ¹(Yₐ¹-R_{b}¹-PG¹)ₘ darstellt, bei der
- Y¹: = entfällt oder O, Ester, Amid, Urethan,
- Rₐ¹: = ein m-wertiger organischer Rest, der 1 bis 30 Kohlenstoffatome und gegebenenfalls auch 0 bis 6 Heteroatome wie O, S oder N enthalten kann,
- m: = 1 bis 3,
- Yₐ¹: = entfällt oder O, Ester, Amid, Urethan,
- R_{b}¹: = entfällt oder ein C₁-C₁₆-Alkylenrest, der durch O-Atome unterbrochen sein kann,
- PG¹: = eine polymerisationsfähige Gruppe, z.B. radikalisch polymerisations-fähige Gruppe, wie (Meth)acrylat, (Meth)acrylamid, Vinyl, Allyl oder Styryl; eine cyclische, radikalisch ringöffnend polymerisierbare Gruppe, wie z.B. die Gruppen 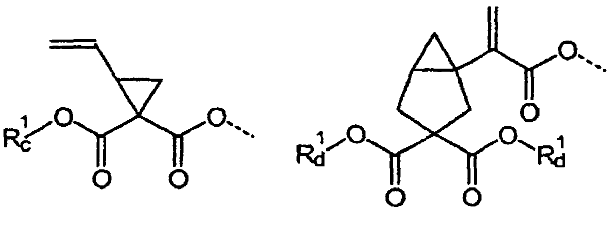 oder eine kationisch polymerisierbare Gruppe, wie z.B. eine Vinylether-, Glycidylgruppe, cycloaliphatische Epoxid- oder Oxetangruppe oder eine polyreaktionsfähige Nitrongruppe. mit
- Y_{b}¹: = entfällt oder O, Ester, Amid, Urethan,
- R_{c}¹, R_{d}¹: = unabhängig voneinander C₁- bis C₁₅-Alkylrest, der durch O unterbrochen sein kann, ein Phenyl-, oder Benzyl-Rest; und mit weiterhin
- X²-X⁴: = unabhängig voneinander entfällt, OH oder C₁- bis C₁₀-Alkylrest und die unabhängig voneinander die gleiche Bedeutung von X¹ haben können und darüber hinaus eine Gruppe der Struktur: (Yₐ²-R_{b}²-HG)ₚ darstellen können, bei der
- Yₐ²: = entfällt oder ggf. O, Ester, Amid, Urethan,
- R_{b}²: = ein p-wertiger organischer Rest, der 1 bis 20 Kohlenstoffatome und gegebenenfalls auch 0 bis 4 Heteroatome wie O oder N enthalten kann,
- p: = 1 bis 3 und
- HG: = eine Haftgruppe wie z.B. -P=O(OH)₂; -O-P=O(OH)₂, -COOH oder -OSO₂OH bedeutet.

Besonders bevorzugt sind polymerisierbare Calix[n]arene entsprechend der allgemeinen Formel (1), bei denen die Variablen der oben gezeigten Gruppen die folgende Bedeutung haben, wobei diese Bedeutungen unabhängig voneinander gewählt werden können:
- n: = 1 bis 3
- R¹-R⁴: = unabhängig voneinander H, ein C₁- bis C₁₀-Alkylrest, der durch O unterbrochen sein kann oder Benzyl-Rest,
- X¹: eine Gruppe der Struktur: Y¹-Rₐ¹(Yₐ¹-R_{b}¹-PG¹)ₘ darstellt, bei der
- Y¹: = entfällt oder O oder Ester,
- Rₐ¹: = ein m-wertiger organischer Rest, der 1 bis 15 Kohlenstoffatome und gegebenenfalls auch 0 bis 3 O-Atome enthalten kann,
- m: = 1 bis 2,
- Yₐ¹: = entfällt oder O oder Ester,
- R_{b}¹: = entfällt oder ein C₁-C₁₆-Alkylenrest, der durch O-Atome unterbrochen sein kann,
- PG¹: = eine polymerisationsfähige Gruppe, z.B. radikalisch polymerisations-fähige Gruppe, wie (Meth)acrylat, (Meth)acrylamid, eine cyclische, radikalisch ringöffnend polymerisierbare Gruppe, wie z.B. die Gruppen 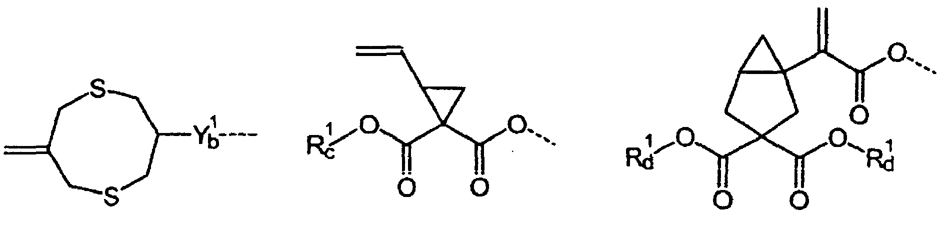 oder eine kationisch polymerisierbare Gruppe, wie z.B. eine cycloaliphatische Epoxid- oder Oxetangruppe oder eine polyreaktionsfähige Nitrongruppe,
mit
- Y_{b}¹: = entfällt oder O, Ester, Urethan,
- R_{c}¹,R_{d}¹: = unabhängig voneinander C₁- bis C₅-Alkylrest, ein Phenyl-, oder Benzyl-Rest;
und mit weiterhin
- X²-X⁴: = entfällt unabhängig voneinander oder C₁- bis C₁₀-Alkylrest und die unabhängig voneinander die gleiche Bedeutung von X¹ haben können und darüber hinaus eine Gruppe der Struktur: (Yₐ²-R_{b}²-HG)ₚ darstellen können, bei der
- Yₐ²: = entfällt oder O oder Ester,
- R_{b}²: = ein p-wertiger organischer Rest, der 1 bis 10 Kohlenstoffatome und gegebenenfalls auch 0 bis 2 O-Atome,
- p: = 1 bis 2 und
- HG: = eine Haftgruppe wie z.B. -P=O(OH)₂; -O-P=O(OH)₂, -COOH oder -O-SO₂OH bedeutet.

Die erfindungsgemäß eingesetzten Calix[n]arene der allgemeinen Formel I können ausgehend von geeignet funktionalisierten Calix[n]arenen durch Umsetzung mit entsprechenden polymerisationsfähigen und säuregruppenhaltigen Verbindungen erhalten werden. So lassen sich z.B. gleichartig substituierte, polymerisationsfähige Calix[n]arene durch Modifizierung von OH-funktionalisierten Calix[n]arenen mit Methacrylsäurechlorid herstellen:

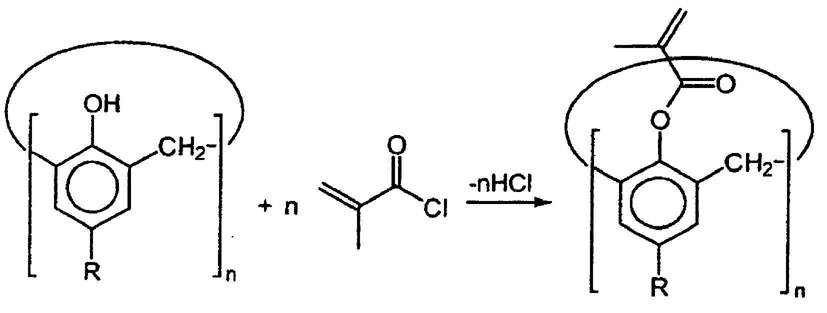

Ein konkretes Beispiel ist die Herstellung des Hexamethacrylates des p-*tert*-Butyl-calix[6]arens:

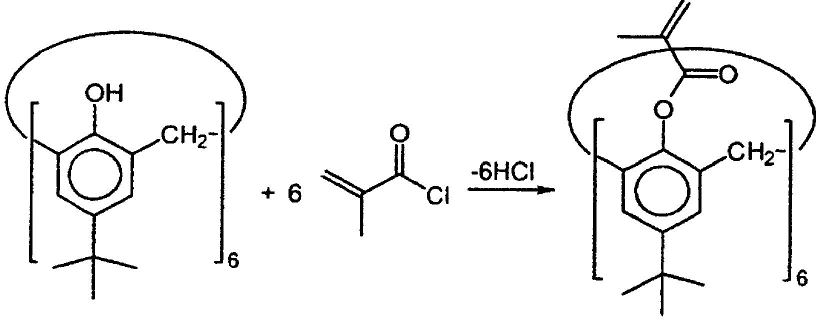

Analog lassen sich ungleichartig substituierte, polymerisationsfähige Calix[n]arene durch Modifizierung von OH-funktionalisierten Calix[n]arenen mit Mischungen von polymerisationsfähigen Säurechloriden, wie z.B. Methacrylsäure- und Acrylsäurechlorid herstellen. Weiterhin können polymerisationsfähige Calix[n]arene, die zusätzliche Säuregruppen tragen durch sequentielle Umsetzung synthestisiert werden, z.B. polymerisationsfähige Calix[n]arene mit Dihydrogenphosphatgruppen durch partielle Umsetzung mit Methacrylsäurechlorid, gefolgt von einer Phosphorylierung mit Phosphoroxychlorid.
Geeignete funktionalisierte Calix[n]arene für die Synthese der erfindungsgemäßen polymerisierbaren Calix[n]arene entsprechend der allgemeinen Formel (1) sind aus der Literatur bekannt, eine Übersicht dazu ist im Review von V. Böhmer (Angew. Chem. 107 (1995) 785-818) zu finden. Danach unterscheidet man einerseits Eintopfverfahren, bei denen z.B. *tert*-Butylphenol mit Formaldehyd unter alkalischen Bedingungen in Abhängigkeit von der Temperatur und der Basenmenge zum Tetra-, Hexa-oder Octamer umgesetzt wird. Andererseits lassen sich unterschiedlich substituierte Calixarene schrittweise durch alternierende Hydroxymethylierungs- und Kondensationsschritte und schließlich nachfolgende Cyclisierung der so erhaltenen linearen Oligomeren synthetisieren.
Geeignete funktionalisierte cyclische Monomere für die Synthese von radikalisch ringöffnend polymerisierbaren Calix[n]arenen der allgemeinen Formel (I) sind aus der Literatur bekannt. Beispielsweise wird die Synthese von Vinylcyclopropanen und von bicyclischen Cyclopropylacrylaten von N. Moszner et al., Macromol. Rapid. Commun. 18 (1997) 775-780, bzw. A. de Meijere et al., Eur. J. Org. Chem, 2004, 3669-3678, beschrieben. Die Synthese von funktionalisierten cyclischen Allylsulfiden wurde von R. A. Evans und E. Rizzardo in J. Polym. Sci., Part A. Polym. Chem. 39 (2001) 202-215; Macromolecules 33 (2000) 6722-6731, publiziert.

Die erfindungsgemäß eingesetzten radikalisch polymerisierbaren Calix[n]arene entsprechend der allgemeinen Formel (I) erlauben die Herstellung von Dentalmaterialien, die sich im Vergleich zu den konventionell auf üblichen Dimethacrylaten basierenden Materialien durch einen geringeren Polymerisationschrumpf und gute mechanische Eigenschaften auszeichnen und darüber hinaus ist z.B. durch die Verwendung von säuregruppenhaltigen, radikalisch polymerisierbaren Calix[n]arenen entsprechend der allgemeinen Formel (I) die Realisierung von weiteren Eigenschaften wie z.B. Selbsthaftung möglich.

Dementsprechend finden die erfindungsgemäßen Materialien als selbsthaftende Dentalmaterialien, z. B. Füllungskomposite, Zemente und Beschichtungsmaterialien Verwendung. Ebenso sind die erfindungsgemäßen Dentalmaterialien als Adhäsive einsetzbar.

Auch wenn die Verwendung der erfindungsgemäßen Zusammensetzungen auf die Verwendung im Dentalbereich gerichtet ist, so haben diese Materialien einen weiten Anwendungsbereich, z. B. lassen sie sich als Schutz- und Abdecklacke für die Optik, Elektronik und die Fahrzeugindustrie verwenden.

Die erfindungsgemäß eingesetzten radikalisch polymerisierbaren Calix[n]arene entsprechend der allgemeinen Formel (1) können in Mischung mit herkömmlichen radikalisch polymerisierbaren Monomeren, insbesondere mit difunktionellen (Meth)acrylat-Vernetzern eingesetzt werden. Diesbezüglich eignen sich vor allem vernetzende bi- oder mehrfunktionelle Acrylate bzw. Methacrylate wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrit-tetra(meth)acrylat, sowie 1,4-Butandioldi(meth)acrylat, 1,10-Decandioldi(meth)acrylat oder 1,12-Dodecandioldi(meth)acrylat.

Besonders vorteilhaft ist die Verwendung der radikalisch polymerisierbaren Calix[n]arene entsprechend der allgemeinen Formel (1) in Mischung mit bekannten schrumpfungsarmen radikalisch ringöffnend polymerisierbaren Monomeren wie z.B. mono- oder multifunktionellen Vinylcyclopropanen bzw. bicyclischen Cyclopropanacrylatderivaten (vgl. DE 196 16 183 C2 bzw. EP 03 022 855) oder cyclischen Allylsulfiden (vgl. US 6,043,361 oder US 6,344,556), die darüber hinaus auch in Kombination mit den voranstehend aufgeführten Di(meth)acrylat-Vernetzern verwendet werden können. Bevorzugte ringöffnend polymerisierbare Monomere sind solche Vinylcyclopropane, wie 1,1-Di(ethoxycarbonyl)- oder 1,1-Di(methoxycarbonyl)-2-vinylcyclopropan oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester oder deren Disubstitutionsprodukte in 3-Stellung wie (3,3-Bis(ethoxycarbonyl)bicyclo[3.1.0]hex-1-yl)acrylsäuremethyl- oder -ethylester. Bevorzugte cyclische Allylsulfide sind vor allem die Additionsprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren Desmodur^{®} VP LS 2294 der Bayer AG.

Besonders geeignet ist die Verwendung der erfindungsgemäßen kationisch polymerisierbaren Calix[n]arene entsprechend der allgemeinen Formel (1) in Mischung mit bekannten schrumpfungsarmen kationisch ringöffnend polymerisierbaren Monomeren wie z.B. Glycidylether oder cycloaliphatische Epoxide, cyclische Ketenacetale, Spiroorthocarbonate, Oxetane oder bicyclische Orthoester. Beispiele sind: 2-Methylen-1,4,6-trioxaspiro[2.2]nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(3,4-epoxycyclohexylmethyl)adipat, Vinylcyclohexendioxid, 3-Ethyl-3-hydroxymethyloxetan, 1,10-Decandiyl-bis-(oxymethylen)-bis-(3-ethyloxetan) oder 3,3-(4-Xylylendioxy)-bis-(methyl-3-ethyloxetan) bzw. weitere in der EP 0 879 257 B1 genannte Epoxide. Als kationisch polymerisierbare Matrixsysteme eignen sich auch Kieselsäurepolykondensate, die beispielsweise durch hydrolytische Kondensation von Silanen, die kationisch polymerisierbare Gruppen, bevorzugt z.B. Epoxid-, Oxetan-, oder Spiroorthoestergruppen tragen. Solche Kieselsäurepolykondensate sind beispielsweise in der DE 41 33 494 C2 oder US 6,096,903 beschrieben.
Die erfindungsgemäßen Dentalmaterialien auf der Basis der radikalisch polymerisierbaren Calix[n]arene entsprechend der allgemeinen Formel (I) lassen sich mit den bekannten radikalischen Initiatoren (vgl. Encyclopedia of Polymer Science and Engineering, Vol. 13, Wiley-Intersci. Pub., New York etc. 1988, 754ff.) polymerisieren. Es eignen sich besonders Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B: Benzoinether, Dialkylbenzilketale, Dialkoxyacetophenone, Acyl- der Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon.
Weiterhin können auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert*-Butylperbenzoat oder Di-(*tert*-butyl)-peroxid verwendet werden. Als Initiatoren für die Heisshärtung eignen sich Benzpinakol und 2,2'-Dialkylbenzpinakole.
Zur Beschleunigung der Initiierung mittels Peroxiden oder α-Diketonen werden auch häufig Kombinationen mit aromatischen Aminen bevorzugt. Redoxsysteme, die sich bereits bewährt haben, sind: Kombinationen aus Benzoylperoxid oder Campherchinon mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und solchen Reduktionsmitteln, wie z.B. Ascorbinsäure, Barbiturate oder Sulfinsäuren, geeignet.
Die erfindungsgemäßen Dentalmaterialien auf der Basis der kationisch polymerisierbaren Calix[n]arene entsprechend der allgemeinen Formel (I) lassen sich mit den bekannten kationischen Photoinitiatoren, besonders mit Diaryliodonium- oder Triarylsulfoniumsalzen, ggf. in Gegenwart geeigneter Sensibilisatoren, wie z.B. Campherchinon, aushärten. Beispiele für geeignete Diaryliodoniumsalze, die mit Campherchinon oder Thioxanthonen als Sensibilisator im sichtbaren Bereich eingesetzt werden können, sind die kommerziell zugänglichen 4-Octyloxy-phenylphenyl-iodoniumhexafluoroantimonat oder Isopropylphenyl-methylphenyl-iodoniumtetrakis(pentafluorophenyl)borat.
Weiterhin können die erfindungsgemäßen Dentalmaterialien auf der Basis der polymerisierbaren Calix[n]arene einen oder mehrere Füllstoffe enthalten, vorzugsweise organische oder anorganische partikuläre Füllstoffe. Bevorzugte anorganische partikulären Füllstoffe sind amorphe kugelförmige nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengröße von 0,2 bis 5 µm sowie röntgenopake Füllstoffe, wie Ytterbiumtrifluorid oder nanopartikuläres Tantal(V)-oxid bzw. Bariumsulfat. Darüber hinaus können auch faserförmige Füllstoffe wie Glasfasern, Polyamid- oder Kohlenstoff-Fasern eingesetzt werden.
Schließlich lassen sich den erfindungsgemäßen Dentalmaterialien auf der Basis der polymerisierbaren Calix[n]arene im Bedarfsfalle weitere Additive zusetzen, wie z.B. Stabilisatoren, UV-Absorber, Farbstoffe oder Pigmente sowie Lösungsmittel, wie z.B. Wasser, Ethanol, Aceton oder Ethylacetat, oder Gleitmittel.
Dabei sind die erfindungsgemäßen Dentalmaterialien je nach Verwendungszweck vorzugsweise aus folgenden Komponenten zusammengesetzt:

Erfindungsgemäße Zemente enthalten bevorzugt:
(a) 0,5 bis 30 Gew.-%, besonders bevorzugt 0,5 bis 20 Gew.-% mindestens eines polymerisierbaren Calix[n]arens gemäß Formel (I),
(b) 0,01 bis 2 Gew.-%, besonders bevorzugt 0,01 bis 1,5 Gew.-% Initiator,
(c) 1 bis 30 Gew.-%, besonders bevorzugt 5 bis 20 Gew.-% mindestens eines weiteren kationisch und/oder radikalisch polymerisierbaren Monomers und/oder eines weiteren ringöffnend polymerisierbaren Monomers, bevorzugt eines mehrfach funktionellen (Meth)acrylats,
(d) 5 bis 70 Gew.-%, besonders bevorzugt 10 bis 60 Gew.-% Füllstoff und
(e) 0,01 bis 5 Gew.-%, bevorzugt 0,01 bis 2 Gew.-%, besonders bevorzugt 0,01 bis 1 Gew.-% Additive,
wobei sich die Prozentangaben jeweils auf 100 % addieren.

Erfindungsgemäße Füllungskomposite enthalten bevorzugt:
(a) 0,5 bis 30 Gew.-%, besonders bevorzugt 0,5 bis 20 Gew.-% mindestens eines polymerisierbaren Calix[n]arens gemäß Formel (I),
(b) 0,01 bis 5 Gew.-%, bevorzugt 0,01 bis 2 Gew.-%, besonders bevorzugt 0,01 bis 1,5 Gew.-% Initiator,
(c) 1 bis 30 Gew.-%, bevorzugt 5 bis 20 Gew.-% besonders bevorzugt 5 bis 15 Gew.-% mindestens eines weiteren kationisch und/oder radikalisch polymerisierbaren Monomers und/oder mindestens eines weiteren ringöffnend polymerisierbaren Monomers, besonders bevorzugt eines mehrfach funktionellen (Meth)acrylats
(d) 5 bis 85 Gew.-%, besonders bevorzugt 10 bis 80 Gew.-% Füllstoff und
(e) 0,01 bis 5 Gew.-%, bevorzugt 0,01 bis 3 Gew.-%, besonders bevorzugt 0,01 bis 2 Gew.-% Additive,
wobei sich die Prozentangaben jeweils auf 100 % addieren.

Erfindungsgemäße Beschichtungsmaterialien enthalten bevorzugt:
(a) 1 bis 70 Gew.-%, besonders bevorzugt 1 bis 50 Gew.-% mindestens eines polymerisierbaren Calix[n]arens gemäß Formel (1),
(b) 0,01 bis 5 Gew.-%,bevorzugt 0,01 bis 2 Gew.-%, besonders bevorzugt 0,1 bis 1,5 Gew.-% Initiator,
(c) 5 bis 70 Gew.-%, bevorzugt 5 bis 60 Gew.-%, besonders bevorzugt 5 bis 50 Gew.-% mindestens eines weiteren kationisch und/oder radikalisch polymerisierbaren Monomers, und/oder mindestens eines weiteren ringöffnend polymerisierbaren Monomers, besonders bevorzugt mindestens eines mehrfach funktionellen (Meth)acrylats,
(d) 1 bis 30 Gew.-%, bevorzugt 3 bis 20 Gew.-%, besonders bevorzugt 3 bis 15 Gew.-% eines Füllstoffs, bevorzugt eines nanopartikulären Füllstoffs und
(e) 0,01 bis 5 Gew.-%, bevorzugt 0,01 bis 3 Gew.-%, besonders bevorzugt 0,01 bis 2 Gew.-%, ganz besonders bevorzugt 0,01 bis 1 Gew.-% Additive
(f) 0 bis 70 Gew.-%, besonders bevorzugt 0 bis 30 Gew.-% Lösungsmittel,
wobei sich die Prozentangaben jeweils auf 100 % addieren.

Erfindungsgemäße Dentaladhäsive enthalten bevorzugt:
(a) 0,5 bis 50 Gew.-%, besonders bevorzugt 1,0 bis 30 Gew.-% mindestens eines polymerisierbaren Calix[n]arens gemäß Formel (I),
(b) 0,01 bis 5 Gew.-%, besonders bevorzugt 0,01 bis 2 Gew.-% mindestens eines Initiators
(c) 5 bis 70 Gew.-%, besonders bevorzugt 5 bis 60 Gew.-% mindestens eines weiteren kationisch und/oder radikalisch polymerisierbaren Monomers und/oder mindestens eines ringöffnend polymersierbaren Monomers, besonders bevorzugt mindestens eines mehrfach funktionellen (Meth)acrylats,
(d) 0 bis 30 Gew.-%, besonders bevorzugt 3 bis 20 Gew.-% Füllstoff und
(e) 0,01 bis 5 Gew.-%, besonders bevorzugt 0,01 bis 3 Gew.-% Additive,
(f) 0 bis 50 Gew.-%, besonders bevorzugt 0 bis 20 Gew.-% Lösungsmittel,
wobei sich die Prozentangaben auf 100 % addieren.

Die Erfindung wird im Folgenden anhand von Beispielen näher erläutert.

### Beispiel 1: Synthese eines Calix[6]arentetramethacrylates V-9

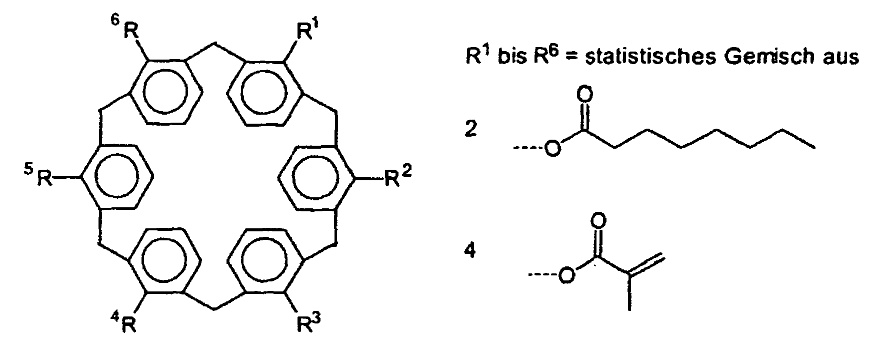

In einem 250 ml Kolben mit Stickstoffhahn werden 9,6 g (15 mmol) Hexahydroxycalix[6]aren und 5,2 g (130 mmol) 60 %iges NaH in Siliconöl in 80 ml trockenem Dimethylformamid gegeben und für 5 Stunden bei RT gerührt. Anschließend werden 5,2 ml (30 mmol) Octansäurechlorid langsam zu der Suspension zugegeben und für weitere 36 h bei RT gerührt. Danach werden langsam 5,8 ml (60 mmol) Methacrylsäurechlorid zugegeben und für weitere 72 h gerührt. Die dunkel gefärbte Suspension wird in 600 ml Wasser gegeben, wobei ein weißer Feststoff ausfällt. Dieser wird filtriert und mit viel Wasser gewaschen. Das Produkt wird aus Toluol und Ethanol umkristallisiert. Die Ausbeute an Produkt V-9 beträgt 8,1 g. Die MALDI-TOF-Analyse des Produktes ergab ein Substitutionsmuster aus 2 Octansäure- und 2-4 Methacrylsäureestergruppen.

### Beispiel 2: Synthese eines Calix[6]arendodecamethacrylates V-15

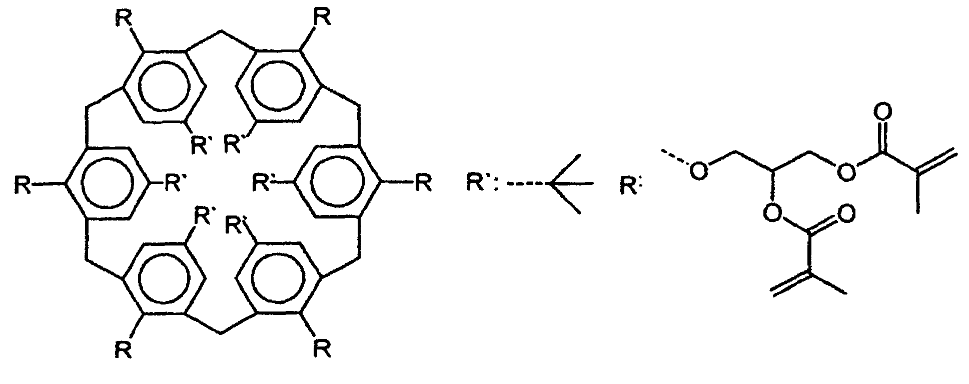

0,98 g p-*tert*-Butylcalix[6]aren (1 mmol), 0,16 g *tert*-Butylammoniumbromid (TBAB) (1,8 mmol) und eine Spatelspitze Phenothiazin werden mit 15 ml N-Methylpyrrolidon (NMP) versetzt und in einen 50 ml Rundkolben gegeben. Anschließend werden der Lösung 4,3 g Glycidylmethacrylat (GMA) (30 mmol) zugesetzt. Der Ansatz wird bei einer Temperatur von 120 °C bei einer Leistung von maximal 100 Watt für 300 min im Mikrowellenfeld gerührt. Die erhaltene klare braune Lösung wird in 500 ml Wasser gefällt, filtriert und schließlich im Hochvakuum getrocknet. Die Ausbeute an Produkt beträgt 1,5 g. Die MALDI-TOF-Analyse des Produktes ergab eine Mischung aus Produkten der 1-6 fachen Umsetzung von p-*tert*-Butylcalix[6]aren mit GMA.
6 g dieses Calix[6]arenderivates werden in 100 ml CH₂Cl₂ gelöst und mit 2,5 g Triethylamin (25 mmol) versetzt und 20 Minuten gerührt. Zu dieser Lösung werden innerhalb 2 Std. 3,1 g Methacrylsäurechlorid (30 mmol) gelöst in 50 ml CH₂Cl₂ bei Raumtemperatur unter Stickstoff zugetropft. Anschließend wird die Reaktionsmischung für weitere 4 Tage gerührt. Die organische Phase wird dann 2-mal mit je 150 ml gesättigter NaHCO₃-Lösung und schließlich mit 400 ml H₂O gewaschen. Die organische Phase wird mit Na₂SO₄ getrocknet und das Lösemittel entfernt. Die Ausbeute an Produkt V-15 beträgt 8,5 g.

### Beispiel 3: Synthese von p-Propyloxy-calix[4]aren-nitron V-10

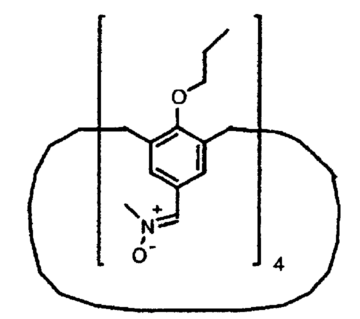

5,11,17,23-Tetraformyl-25,26,27,28-tetrapropoxycalix[4]aren (4.5 mmol) und *N-*Methylhydroxylaminhydrochlorid (27 mmol) werden in 60 ml Ethanol/NaOH (27 mmol)-Lösung suspendiert und bei Raumtemperatur unter N₂-Atmosphere gerührt. Nachdem eine komplette Umsetzung erreicht ist (FT-IR), wird die Reaktion beendet. Anschließend wird das Lösungsmittel am Rotationsverdampfer entfernt. Das Rohprodukt wird mit Wasser/Chloroform (je 100 ml) ausgeschüttelt. Die trübe organische Phase wird abgetrennt, filtriert und anschließend das Lösungsmittel am Rotationsverdampfer entfernt. Das Produkt wird im Ölpumpen-Vakuum getrocknet. Kleinste Mengen an Verunreinigung werden durch Säulenchromatographie mit Methanol abgetrennt. Die Ausbeute an Produkt V-1 0 beträgt ca. 100%.

### Beispiel 4: Herstellung eines Kompositzementes auf der Basis des polymerisierbaren Calix[6]arens V-15 aus Beispiel 2

Entsprechend der nachfolgend aufgeführten Tabelle 1 wurde ein Kompositbefestigungszement auf der Basis einer Methacrylatmischung (Material A, Vergleich) und unter Einbeziehung des Calix[6]arens V-15 aus Beispiel 2 (Material B) mittels eines Walzenstuhles "Exakt" (Exakt Apparatebau, Norderstedt) hergestellt. Von den Materialien wurden entsprechende Prüfkörper präpariert, die 2 mal 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls.

**Tabelle 1: Zusammensetzung Kompositzement (Angaben in Gew.-%)**

| Stoffe | Material A | Material B |
|---|---|---|
| Triethylenglycoldimethacrylat | 39,6 | 31,8 |
| Calix[6]aren V-15 aus Beispiel 2 | - | 7,8 |
| Aerosil OX-50 (Degussa) | 41,3 | 41,3 |
| Ytterbiumtrifluorid (Rhone-Poulenc) | 18,7 | 18,7 |
| Photoinitiator¹⁾ | 0,4 | 0,4 |

| | | |
|---|---|---|
| ¹⁾ Mischung aus Campherchinon (0,24 Gew.-%), p-N,N-Dimethylaminobenzoesäure-ethylester (0,26 Gew.-%) | | |

Aus Tabelle 2 ist ersichtlich, dass das Material B im Vergleich zum Material A (auf der Basis einer rein konventionellen Methacrylatmischung) zumindest zu vergleichbaren mechanischen Eigenschaften führt.

**Tabelle 2: Zementeigenschaften**

| Materialeigenschaft | Material A | Material B |
|---|---|---|
| Biegefestigkeit (MPa) nach 24 h | 77 | 58 |
| Biegefestigkeit (MPa) nach 24 h WL¹⁾ | 71 | 62 |
| Biege-E-Modul (GPa) nach 24 h | 4,32 | 4,20 |
| Biege-E-Modul (GPa) nach 24 h WL | 4,10 | 4,00 |

| | | |
|---|---|---|
| ¹⁾ WL = Wasserlagerung der Prüfkörper bei 37 °C | | |

### Beispiel 5: Herstellung eines Füllungskomposites auf der Basis des polymerisierbaren Calix[6]arens V-15 aus Beispiel 2

Entsprechend der nachfolgend aufgeführten Tabelle 3 wurde ein Füllungskomposit auf der Basis einer Methacrylatmischung (Material C, Vergleich) und unter Einbeziehung des Calix[6]arens V-15 aus Beispiel 2 (Material D) mittels eines Kneters LPM 0.1 SP (Linden, Marienheide) hergestellt. Von den Materialien wurden analog Beispiel 3 Prüfkörper hergestellt und ausgehärtet. Nach der ISO-Norm ISO-4049 erfolgte die Bestimmung der Biegefestigkeit, des Biege-E-Moduls und des Polymerisationsschrumpfes.

**Tabelle 3: Zusammensetzung Füllungskomposit (Angaben in Gew.-%)**

| Stoffe | Material C | Material D |
|---|---|---|
| Tetric-Monomer¹) | 18,1 | 16,3 |
| Calix[6]aren V-15 aus Beispiel 2 | - | 1.8 |
| Glasfüller GM27884 (Degussa)²⁾ | 52,2 | 52,2 |
| Sphärosil (Tokoyama Soda)³⁾ | 14,5 | 14,5 |
| Ytterbiumtrifluorid (Rhone-Poulenc) | 15,0 | 15,0 |
| Photoinitiator⁴⁾ | 0,2 | 0,2 |

| | | |
|---|---|---|
| ¹⁾ Mischung aus 42.4 Gew.-% Bis-GMA, 37,4 Gew.-% UDMA und 20,2 Gew.-% Triethylenglycoldimethacrylat ²⁾ Silanisierter Ba-Al-Borosilikatglasfüller mit einer mittleren Partikelgrösse von 1,5 µm, ³⁾ SiO₂-ZrO₂-Mischoxid (mittlere Primärpartikelgrösse: 250 nm) ⁴⁾ Mischung aus Campherchinon (0,24 Gew.-%), p-N,N-Dimethylaminobenzoesäureethylester (0,26 Gew.-%) | | |

**Tabelle 4: Füllungskompositeigenschaften**

| Materialeigenschaft | Material C | Material D |
|---|---|---|
| Biegefestigkeit (MPa) nach 24 h | 140 | 121 |
| Biegefestigkeit (MPa) nach 24 h WL¹⁾ | 163 | 150 |
| Biege-E-Modul (GPa) nach 24 h | 11,4 | 11,2 |
| Biege-E-Modul (GPa) nach 24 h WL | 11,8 | 10,9 |
| Polymerisationsschrumpf (Vol.-%) | -3.98 | -3,22 |

| | | |
|---|---|---|
| ¹⁾ WL = Wasserlagerung der Prüfkörper bei 37 °C | | |

Aus Tabelle 4 ist ersichtlich, dass das Material D im Vergleich zum Material C (auf der Basis einer rein konventionellen Methacrylatmischung) bei vergleichbaren mechanischen Eigenschaften zu einem signifikant verringerten Polymerisationsschrumpf führt.

### Beispiel 6: Herstellung eines Füllungskomposites auf der Basis des polymerisierbaren Calix[6]arens V-9 aus Beispiel 1

Mit den Inhaltstoffen in Tabelle 5 wurde ein Füllungskomposit auf der Basis einer Methacrylatmischung (Material E, Vergleich) und unter Einbeziehung des Calix[6]arens V-9 aus Beispiel 1 (Material F) mittels eines Kneters VPL 1.5 (Grieser, Lampertheim) hergestellt. Von den Materialien wurden normgerechte Prüfkörper hergestellt und ausgehärtet. Nach der ISO-Norm ISO-4049 erfolgte die Bestimmung der Biegefestigkeit, des Biege-E-Moduls und des Polymerisationsschrumpfes bzw. der Polymerisationsschrumpfkraft.

**Tabelle 5: Zusammensetzung Füllungskomposit (Angaben in Gew.-%)**

| Stoffe | Material E | Material F |
|---|---|---|
| TEGDMA¹⁾ | 9,1 | 7,8 |
| Bis-GMA | 9,1 | 9,1 |
| Calix[6]aren V-9 aus Beispiel 1 | - | 1,3 |
| Glasfüller GM018-053 (Schott)²⁾ | 76,0 | 76,0 |
| Nano-SiO₂ ³⁾ | 5,0 | 5,0 |
| Stabilisatoren | 0,3 | 0,3 |
| Photoinitiator⁴⁾ | 0,5 | 0,5 |

| | | |
|---|---|---|
| ¹⁾ Triethylenglycoldimethacrylat ²⁾ Silanisierter Ba-Al-Borosilikatglasfüller mit einer mittleren Partikelgrösse von 0,7 µm, ³⁾ SiO₂-Dispersion (mittlere Partikelgrösse: 20 nm) ⁴⁾ Mischung aus Campherchinon (0,36 Gew.-%), p-N,N-Dimethylaminobenzoesäureethylhexylester (0,14 Gew.-%) | | |

**Tabelle 6: Füllungskompositeigenschaften**

| Materialeigenschaft | Material E | Material F |
|---|---|---|
| Biegefestigkeit (MPa) nach 24 h WL¹⁾ | 116 | 103 |
| Biege-E-Modul (GPa) nach 24 h WL | 7,8 | 7,9 |
| Polymerisationsschrumpf (Vol.-%) | -2,4 | -1,8 |
| Polymerisationsschrumpfkraft (MPa) nach 24 h WL | 5,9 | 5,4 |

| | | |
|---|---|---|
| ¹⁾ WL = Wasserlagerung der Prüfkörper bei 37 °C | | |

Aus Tabelle 6 ist ersichtlich, dass das Material F im Vergleich zum Material E (auf der Basis einer rein konventionellen Methacrylatmischung) bei vergleichbaren mechanischen Eigenschaften zu einem signifikant verringerten Polymerisationsschrumpf führt.

### Beispiel 7: Herstellung eines Füllungskomposites auf der Basis des vernetzbaren Calix[4]arens V-10 aus Beispiel 3

Mit den Inhaltstoffen in Tabelle 6 wurde ein Füllungskomposit auf der Basis einer Methacrylatmischung (Material E, Vergleich) und unter Einbeziehung des Calix[4]arens V-10 aus Beispiel 3 (Material G) mittels eines Kneters VPL 1.5 (Grieser, Lampertheim) hergestellt. Von den Materialien wurden normgerechte Prüfkörper hergestellt und ausgehärtet. Nach der ISO-Norm ISO-4049 erfolgte die Bestimmung der Biegefestigkeit, des Biege-E-Moduls und des Polymerisationsschrumpfes bzw. der Polymerisationsschrumpfkraft.

**Tabelle 7: Zusammensetzung Füllungskomposit (Angaben in Gew.-%)**

| Stoffe | Material E | Material G |
|---|---|---|
| TEGDMA¹⁾ | 9,1 | 7,8 |
| Bis-GMA | 9,1 | 9,1 |
| Calix[6]aren V-9 aus Beispiel 1 | - | 1,3 |
| Glasfüller GM018-053 (Schott)²⁾ | 76,0 | 76,0 |
| Nano-SiO₂ ³⁾ | 5,0 | 5,0 |
| Stabilisatoren | 0,3 | 0,3 |
| Photoinitiator⁴⁾ | 0,5 | 0,5 |

| | | |
|---|---|---|
| ¹⁾ Triethylenglycoldimethacrylat ²⁾ Silanisierter Ba-Al-Borosilikatglasfüller mit einer mittleren Partikelgrösse von 0,7 µm, ³⁾ SiO₂-Dispersion (mittlere Partikelgrösse: 20 nm) ⁴⁾ Mischung aus Campherchinon (0,36 Gew.-%), p-N,N-Dimethylaminobenzoesäureethylhexylester (0,14 Gew.-%) | | |

**Tabelle 8: Füllungskompositeigenschaften**

| Materialeigenschaft | Material E | Material G |
|---|---|---|
| Biegefestigkeit (MPa) nach 24 h WL¹⁾ | 116 | 90 |
| Biege-E-Modul (GPa) nach 24 h WL | 7,8 | 6,5 |
| Polymerisationsschrumpf (Vol.-%) | -2,4 | -1,7 |
| Polymerisationsschrumpfkraft (MPa) nach 24 h WL | 5,9 | 4,6 |

| | | |
|---|---|---|
| ¹⁾ WL = Wasserlagerung der Prüfkörper bei 37 °C | | |

Aus Tabelle 8 ist ersichtlich, dass das Material G im Vergleich zum Material E (auf der Basis einer rein konventionellen Methacrylatmischung) bei wenig reduzierten mechanischen Eigenschaften zu einem signifikant verringerten Polymerisationsschrumpf führt.

## Patentansprüche

1. Verwendung von Zusammensetzungen aus den Komponenten
(a) 0,5 bis 90 Gew.-% mindestens eines polymerisierbaren Calix[n]arens gemäß Formel (I), 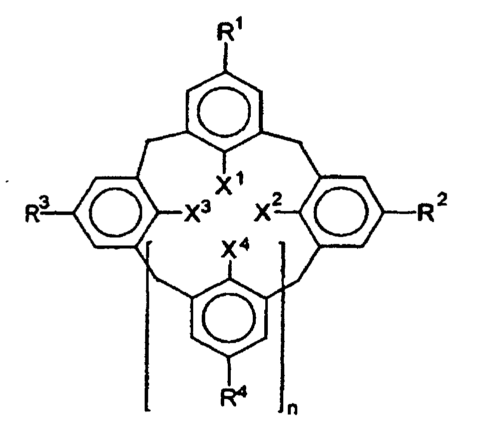 wobei
n = 1 bis 5
R¹-R⁴ = unabhängig voneinander H, ein C₁- bis C₁₅-Alkylrest, der durch O unterbrochen sein kann, ein Phenyl-, oder Benzyl- Rest,
X¹ eine Gruppe der Struktur. Y¹-Rₐ¹(Yₐ¹-R_{b}¹-PG¹)ₘ darstellt, bei der
Y¹ = entfällt oder O, Ester, Amid, Urethan,
Rₐ¹ = ein m-wertiger organischer Rest, der 1 bis 30 Kohlenstoffatome und gegebenenfalls auch 0 bis 6 Heteroatome wie O, S oder N enthalten kann,
m = 1 bis 3,
Yₐ¹ = entfällt oder O, Ester, Amid, Urethan,
R_{b}¹ entfällt oder ein C₁-C₁₆-Alkylenrest, der durch O-Atome unterbrochen sein kann,
PG¹ = eine polymerisationsfähige Gruppe, z.B. radikalisch polymerisationsfähige Gruppe, wie (Meth)acrylat, (Meth)acrylamid, Vinyl, Allyl oder Styryl; eine cyclische, radikalisch ringöffnend polymerisierbare Gruppe, wie z.B. die Gruppen 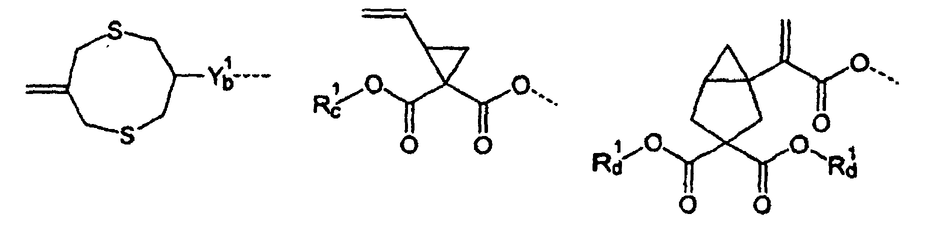 oder eine kationisch polymerisierbare Gruppe, wie z.B. eine Vinylether-, Glycidylgruppe, cycloaliphatische Epoxid- oder Oxetangruppe bzw. polyreaktionsfähige Nitrongruppe, mit
Y_{b}¹ = entfällt oder O, Ester, Amid, Urethan,
R_{c}¹,R_{d}¹ = unabhängig voneinander C₁- bis C₁₅-Alkylrest, der durch O unterbrochen sein kann, ein Phenyl-, oder Benzyl-Rest; und mit weiterhin
X²-X⁴ = unabhängig voneinander entfällt, OH oder C₁- bis C₁₀- Alkylrest und die unabhängig voneinander die gleiche Bedeutung von X¹ haben können und darüber hinaus eine Gruppe der Struktur: (Yₐ²-R_{b}²-HG)ₚ darstellen können, bei der
Yₐ² = entfällt oder O, Ester, Amid, Urethan,
R_{b}² = ein p-wertiger organischer Rest, der 1 bis 20 Kohlenstoffatome und gegebenenfalls auch 0 bis 4 Heteroatome wie O oder N enthalten kann,
p = 1 bis 3 und
HG = eine Haftgruppe wie -P=O(OH)2; -O-P=O(OH)2, -COOH oder -O-SO2OH bedeutet
(b) 0,01 bis 5 Gew.-% Initiator,
(c) 0 bis 90 Gew.-% mindestens eines weiteren kationisch und/oder radikalisch polymerisierbaren Monomers und/oder ringöffnend polymerisierbaren Monomers,
(d) 0 bis 85 Gew.-% Füllstoff und
(e) 0,01 bis 5 Gew.-% Additive,
wobei sich die Prozentangaben jeweils auf 100 % addieren, zur Herstellung von Dentalmaterialien.

2. Verwendung nach Anspruch 1 enthaltend
(a) 0,5 bis 40 Gew.-% mindestens eines Calix[n]arens gemäß Formel (I),
(b) 0,01 bis 2 Gew.-% Initiator,
(c) 1 bis 70 Gew.-% eines weiteren kationisch und/oder radikalisch polymerisierbaren und/oder ringöffnend polymerisierbaren Monomers,
(d) 3 bis 80 Gew.%. Füllstoff und
(e) 0,01 bis 3 Gew.-% Additive,
wobei sich die Prozentangaben jeweils auf 100 % addieren.

3. Verwendung nach Anspruch 2 enthaltend Calix[n]arene der Formel (I): 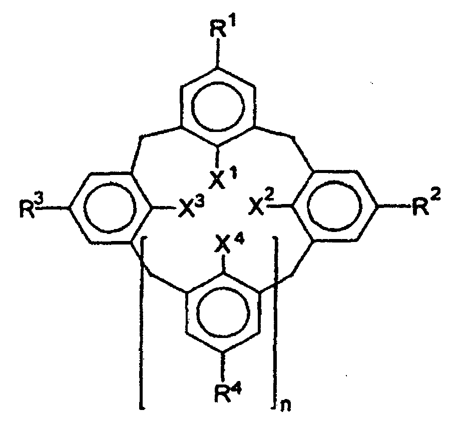 wobei
n = 1 bis 3
R¹-R⁴ = unabhängig voneinander H, ein C₁- bis C₁₀-Alkylrest, der durch O unterbrochen sein kann oder Benzyl-Rest,
X¹ eine Gruppe der Struktur: Y¹-Rₐ¹(Yₐ¹-R_{b}¹-PG¹)ₘ darstellt, bei der
Y¹ = entfällt oder O oder Ester,
Rₐ¹ = ein m-wertiger organischer Rest, der 1 bis 15 Kohlenstoffatome und gegebenenfalls auch 0 bis 3 O- Atome enthalten kann,
m = 1 bis 2,
Yₐ¹ = entfällt oder O oder Ester,
R_{b}¹ = entfällt oder ein C₁-C₁₆-Alkylenrest, der durch O-Atome unterbrochen sein kann,
PG¹ = eine polymerisationsfähige Gruppe, radikalisch polymerisationsfähige Gruppe, wie (Meth)acrylat, (Meth)acrylamid, eine cyclische, radikalisch ringöffnend polymerisierbare Gruppe, wie z.B. die Gruppen 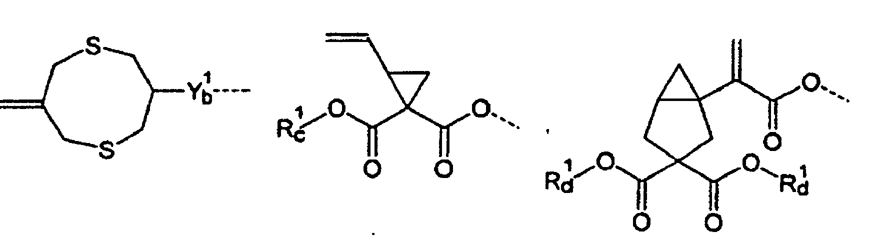 oder eine kationisch polymerisierbare Gruppe, wie z.B. eine cycloaliphatische Epoxid- oder Oxetangruppe oder eine polyreaktionsfähige Nitrongruppe, mit
Y_{b}¹ = entfällt oder O, Ester, Urethan,
R_{c}¹,R_{d}¹ = unabhängig voneinander C₁- bis C₅-Alkylrest, ein Phenyl-, oder Benzyl-Rest;
und mit weiterhin
X²-X⁴ = unabhängig voneinander entfällt oder C₁- bis C₁₀-Alkylrest und die unabhängig voneinander die gleiche Bedeutung von X¹ haben können und darüber hinaus eine Gruppe der Struktur: (Yₐ²-R_{b}²-HG)ₚ darstellen können, bei der
Yₐ² = entfällt oder O oder Ester,
R_{b}² = ein p-wertiger organischer Rest, der 1 bis 10 Kohlenstoffatome und gegebenenfalls auch 0 bis 2 O- Atome,
p = 1 bis 2 und
HG = eine Haftgruppe wie z.B. -P=O(OH)₂; -O-P=O(OH)₂, -COOH oder -O-SO₂OH bedeutet.

4. Verwendung nach einem der vorhergehenden Ansprüche **dadurch gekennzeichnet, daß** als radikalisch polymerisierbare Monomere ringöffnend polymerisierbare Monomere, insbesondere mono- oder multifunktionelle Vinylcyclopropane bzw. bicyclische Cylopropanacrytate oder cyclische Allylsulfide und mehrfach funktionelle (Meth)acrylate oder Gemisch dieser Monomere enthalten sind.

5. Verwendung nach einem der vorhergehenden Ansprüche, **dadurch gekennzeichnet, daß** die Zusammensetzungen Zemente sind, enthaltend
(a) 0,5 bis 30 Gew.-% mindestens eines Calix[n]arens gemäß Formel (1),
(b) 0,01 bis 2 Gew.-% Initiator,
(c) 1 bis 30 Gew.-% mindestens eines weiteren kationisch und/oder radikalisch polymerisierbaren Monomers, und/oder mindestens eines weiteren ringöffnend polymerisierbaren Monomers,
(d) 5 bis 70 Gew.-% Füllstoff und
(e) 0,01 bis 5 Gew.-% Additive,
wobei sich die Prozentangaben jeweils auf 100 % addieren.

6. Verwendung nach Anspruch 5 **dadurch gekennzeichnet, dass** die Zusammensetzungen
(a) 0,5 bis 20 Gew.-% mindestens eines Calix[n]arens gemäß Formel (I),
(b) 0,01 bis 1,5 Gew.-% Initiator,
(c) 5 bis 20 Gew.-% mindestens eines weiteren radikalisch polymerisierbaren Monomers, und/oder mindestens eines weiteren ringöffnend polymerisierbaren Monomers,
(d) 10 bis 60 Gew.-% Füllstoff und
(e) 0,01 bis 3 Gew.-% Additive
enthalten, wobei sich die Prozentangaben jeweils auf 100 % addieren.

7. Verwendung nach einem der Ansprüche 1 bis 4, **dadurch gekennzeichnet, daß** die Zusammensetzungen Füllungskomposite sind, enthaltend
(a) 0,5 bis 30 Gew.-% mindestens eines Calix[n]arens gemäß Formel (I),
(b) 0,01 bis 5 Gew.-% Initiator,
(c) 1 bis 30 Gew.-% mindestens eines weiteren ionisch oder radikalisch polymerisierbaren Monomers, und/oder mindestens eines weiteren ringöffnend polymerisierbaren Monomers,
(d) 5 bis 85 Gew.-% Füllstoff und
(e) 0,01 bis 5 Gew.-% Additive,
wobei sich die Prozentangaben jeweils auf 100 % addieren.

8. Verwendung nach Anspruch 7, **dadurch gekennzeichnet, daß** die Zusammensetzungen
(a) 0,5 bis 20 Gew.-% mindestens eines Calix[n]arens gemäß Formel (I),
(b) 0,01 bis 2 Gew.-% Initiator,
(c) 5 bis 20 Gew.-% mindestens eines weiteren radikalisch polymerisierbaren Monomers, und/oder mindestens eines weiteren ringöffnend polymerisierbaren Monomers,
(d) 10 bis 80 Gew.-% Füllstoff und
(e) 0,01 bis 3 Gew.-% Additive
enthalten, wobei sich die Prozentangaben jeweils auf 100 % addieren.

9. Verwendung nach einem der Ansprüche 1 bis 4, **dadurch gekennzeichnet, daß** die Zusammensetzungen Beschichtungsmaterialien sind, enthaltend
(a) 0,5 bis 70 Gew.-% mindestens eines Calix[n]arens gemäß Formel (I),
(b) 0,01 bis 5 Gew.-% Initiator,
(c) 5 bis 60 Gew.-% mindestens eines weiteren ionisch oder radikalisch polymerisierbaren Monomers und/oder mindestens eines weiteren ringöffnend polymerisierbaren Monomers,
(d) 1 bis 30 Gew.-% eines Füllstoffs
(e) 0,01 bis 5 Gew.-% Additive und
(f) 0 bis 70 Gew.-% Lösungsmittel,
wobei sich die Prozentangaben jeweils auf 100 % addieren.

10. Verwendung nach Anspruch 9, **dadurch gekennzeichnet**, **dadurch gekennzeichnet, daß** die Zusammensetzungen
(a) 1 bis 50 Gew.-% mindestens eines Calix[n]arens gemäß Formel (I),
(b) 0,01 bis 1,5 Gew.-% Initiator,
(c) 5 bis 60 Gew.-% mindestens eines weiteren radikalisch polymerisierbaren Monomers, und/oder mindestens eines weiteren ringöffnend polymerisierbaren Monomers,
(d) 3 bis 20 Gew.-% eines Füllstoffs,
(e) 0,01 bis 3 Gew.-% Additive und
(f) 0 bis 30 Gew.-% Lösungsmittel,
enthalten, wobei sich die Prozentangaben jeweils auf 100 % addieren.

11. Verwendung nach einem der Ansprüche 1 bis 4 **dadurch gekennzeichnet, dass** die Zusammensetzungen Dentaladhäsive sind, enthaltend
(a) 0,5 bis 50 Gew.-% mindestens eines Calix[n]arens gemäß Formel (I)
(b) 0,01 bis 5 Gew.-% mindestens eines Initiators
(c) 5 bis 70 Gew.-% mindestens eines weiteren radikalisch polymerisierbaren Monomers und/oder mindestens eines weiteren ringöffnend polymerisierbaren Monomers
(d) 0 bis 30 Gew.-% Füllstoff
(e) 0,01 bis 5 Gew.-% Additive und
(f) 0 bis 50 Gew.-% Lösungsmittel
wobei sich die Prozentangaben jeweils auf 100 % addieren.

12. Verwendung nach Anspruch 11 **dadurch gekennzeichnet, dass** die Zusammensetzungen
(a) 1 bis 30 Gew.-% eines Calix[n]arens gemäß Formel (I)
(b) 0,01 bis 2 Gew.-% mindestens eines Initiators
(c) 5 bis 60 Gew.-%, mindestens eines weiteren kationisch und/oder radikalisch und/oder ringöffnend polymerisierbaren Monomers
(d) 3 bis 20 Gew.-% Füllstoff
(e) 0,01 bis 3 Gew.-% Additive und
(f) 0 bis 20 Gew.-% Lösungsmittel,
enthalten, wobei sich die Prozentangaben auf 100% addieren.

## Claims

1. Use of compositions consisting of the components
(a) 0.5 to 90% by weight of at least one polymerizable calix[n]arene according to formula (I), 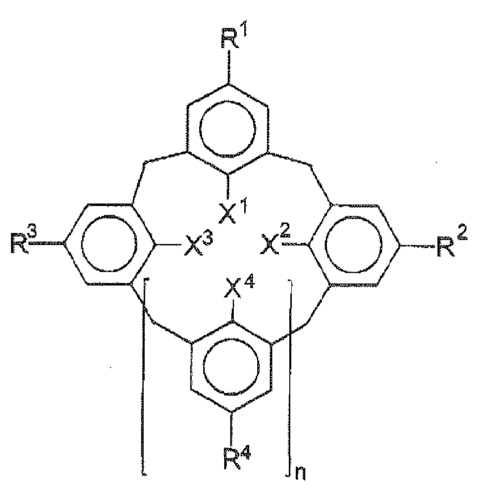 in which
n = 1 to 5,
R¹-R⁴ = independently of one another, H, a C₁- to C₁₅- alkyl radical which can be interrupted by O, a phenyl radical or a benzyl radical,
X¹ represents a group with the structure: Y¹-Rₐ¹(Yₐ¹-R_{b}¹-PG¹)ₘ, in which
Y¹ = not present or O, ester, amide or urethane,
Rₐ¹ = an m-valent organic radical which can comprise from 1 to 30 carbon atoms and, if appropriate, also from 0 to 6 heteroatoms, such as O, S or N,
m = 1 to 3,
Yₐ¹ = not present or O, ester, amide or urethane,
R_{b}¹ = not present or a C₁-C₁₆-alkylene radical which can be interrupted by oxygen atoms,
PG¹ = a polymerizable group, a group which can polymerize under radical conditions, such as (meth)acrylate, (meth)acrylamide, vinyl, allyl or styryl; a cyclic group which can polymerize under radical conditions by ring opening, such as, e.g., the groups 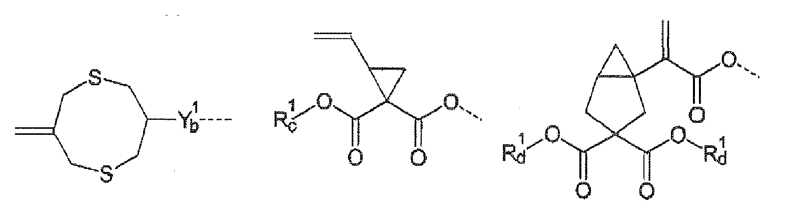 Y_{b}¹ or a group which can polymerize under cationic conditions, such as, e.g., a vinyl ether or glycidyl group, a cycloaliphatic epoxide or oxetane group or a polymerizable nitrone group, with = not present or O, ester, amide or urethane,
R_{c}¹, R_{d}¹ = independently of one another, C₁- to C₁₅-alkyl radical which can be interrupted by O, a phenyl radical or a benzyl radical; and with furthermore
X²-X⁴ = independently of one another, not present, OH or C₁- to C₁₀-alkyl radical and which can have, independently of one another, the same meaning of X¹ and, in addition, can represent a group with the structure: (Yₐ²-R_{b}²-AG)ₚ, in which
Yₐ² = not present or O, ester, amide or urethane, R_{b}² = a p-valent organic radical which can comprise from 1 to 20 carbon atoms and, if appropriate, also from 0 to 4 heteroatoms, such as O or N,
p = 1 to 3 and
AG = an anchoring group, such as -P=O(OH)₂, -O-P=O(OH)₂, -COOH or -O-SO₂OH,
(b) 0.01 to 5% by weight of initiator,
(c) 0 to 90% by weight of at least one additional monomer which can polymerize under cationic and/or radical conditions and/or monomer which can polymerize by ring opening,
(d) 0 to 85% by weight of filler and
(e) 0.01 to 5% by weight of additives, the percentages each time adding up to 100%, for producing dental materials.

2. Use according to Claim 1, comprising
(a) 0.5 to 40% by weight of at least one calix[n]arene according to formula (I),
(b) 0.01 to 2% by weight of initiator,
(c) 1 to 70% by weight of an additional monomer which can polymerize under cationic and/or radical conditions and/or which can polymerize by ring opening,
(d) 3 to 80% by weight of filler and
(e) 0.01 to 3% by weight of additives,
the percentages each time adding up to 100%.

3. Use according to Claim 2, comprising calix[n]arenes of the formula (I): 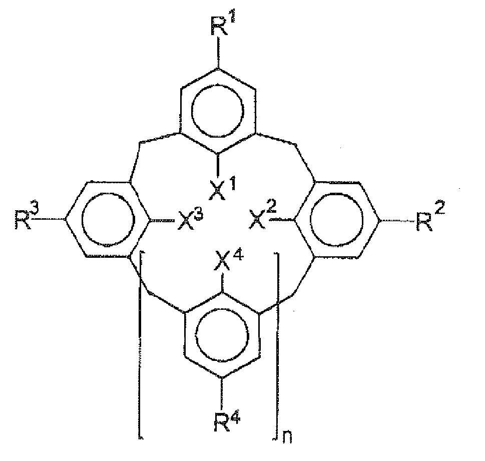 in which
n = 1 to 3,
R¹-R⁴ = independently of one another, H, a C₁- to C₁₀- alkyl radical which can be interrupted by O, or a benzyl radical,
X¹ represents a group with the structure: Y¹-Rₐ¹(Yₐ¹-R_{b}¹-PG¹)ₘ, in which
Y¹ = not present or O or ester,
Rₐ¹ = an m-valent organic radical which can comprise from 1 to 15 carbon atoms and, if appropriate, also from 0 to 3 oxygen atoms,
m = 1 to 2,
Yₐ¹ = not present or O or ester,
R_{b}¹ = not present or a C₁-C₁₆-alkylene radical which can be interrupted by oxygen atoms,
PG¹ = a polymerizable group, e.g., a group which can polymerize under radical conditions, such as (meth)acrylate or (meth)acrylamide, a cyclic group which can polymerize under radical conditions by ring opening, such as, e.g., the groups 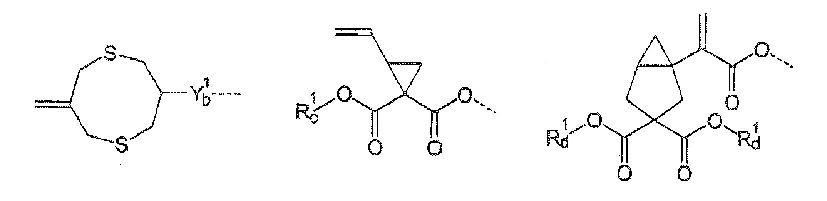 or a group which can polymerize under cationic conditions, such as, e.g., a cycloaliphatic epoxide or oxetane group or a polymerizable nitrone group, with
Y_{b}¹ = not present or O, ester or urethane,
R_{c}¹, R_{d}¹ = independently of one another, C₁- to C₅-alkyl radical, a phenyl radical or a benzyl radical;
and with furthermore
X²-X⁴ = independently of one another, not present or C₁- to C₁₀-alkyl radical and which can have, independently of one another, the same meaning of X¹ and, in addition, can represent a group with the structure: (Yₐ²-R_{b}²-AG)ₚ, in which
Yₐ² = not present or O or ester,
R_{b}² = a p-valent organic radical which can comprise from 1 to 10 carbon atoms and, if appropriate, also from 0 to 2 oxygen atoms,
p = 1 to 2 and
AG = an anchoring group, such as, e.g., -P=O(OH)₂, -O-P=O(OH)₂, -COOH or -O-SO₂OH.

4. Use according to any of the preceding claims, **characterized in that** there are present, as monomers which can polymerize under radical conditions, monomers which can polymerize by ring opening, in particular mono- or polyfunctional vinylcyclopropanes or bicyclic cyclopropaneacrylates or cyclic allyl sulphides, and polyfunctional (meth)acrylates or mixtures of these monomers.

5. Use according to any of the preceding claims, **characterized in that** the compositions are cements comprising
(a) 0.5 to 30% by weight of at least one calix[n]arene according to formula (I),
(b) 0.01 to 2% by weight of initiator,
(c) 1 to 30% by weight of at least one additional monomer which can polymerize under cationic and/or radical conditions and/or at least one additional monomer which can polymerize by ring opening,
(d) 5 to 70% by weight of filler and
(e) 0.01 to 5% by weight of additives,
the percentages each time adding up to 100%.

6. Use according to Claim 5, **characterized in that** the compositions comprise
(a) 0.5 to 20% by weight of at least one calix[n]arene according to formula (I),
(b) 0.01 to 1.5% by weight of initiator,
(c) 5 to 20% by weight of at least one additional monomer which can polymerize under radical conditions and/or at least one additional monomer which can polymerize by ring opening,
(d) 10 to 60% by weight of filler and
(e) 0.01 to 3% by weight of additives,
the percentages each time adding up to 100%.

7. Use according to any of Claims 1 to 4, **characterized in that** the compositions are filling composites comprising
(a) 0.5 to 30% by weight of at least one calix[n]arene according to formula (I),
(b) 0.01 to 5% by weight of initiator,
(c) 1 to 30% by weight of at least one additional monomer which can polymerize under ionic or radical conditions and/or at least one additional monomer which can polymerize by ring opening,
(d) 5 to 85% by weight of filler and
(e) 0.01 to 5% by weight of additives,
the percentages each time adding up to 100%.

8. Use according to Claim 7, **characterized in that** the compositions comprise
(a) 0.5 to 20% by weight of at least one calix[n]arene according to formula (I),
(b) 0.01 to 2% by weight of initiator,
(c) 5 to 20% by weight of at least one additional monomer which can polymerize under radical conditions and/or at least one additional monomer which can polymerize by ring opening,
(d) 10 to 80% by weight of filler and
(e) 0.01 to 3% by weight of additives,
the percentages each time adding up to 100%.

9. Use according to any of Claims 1 to 4, **characterized in that** the compositions are coating materials comprising
(a) 0.5 to 70% by weight of at least one calix[n]arene according to formula (I),
(b) 0.01 to 5% by weight of initiator,
(c) 5 to 60% by weight of at least one additional monomer which can polymerize under ionic or radical conditions and/or at least one additional monomer which can polymerize by ring opening,
(d) 1 to 30% by weight of a filler,
(e) 0.01 to 5% by weight of additives and
(f) 0 to 70% by weight of solvent,
the percentages each time adding up to 100%.

10. Use according to Claim 9, **characterized in that** the compositions comprise
(a) 1 to 50% by weight of at least one calix[n]arene according to formula (I),
(b) 0.01 to 1.5% by weight of initiator,
(c) 5 to 60% by weight of at least one additional monomer which can polymerize under radical conditions and/or at least one additional monomer which can polymerize by ring opening,
(d) 3 to 20% by weight of a filler,
(e) 0.01 to 3% by weight of additives and
(f) 0 to 30% by weight of solvent,
the percentages each time adding up to 100%.

11. Use according to any of Claims 1 to 4, **characterized in that** the compositions are dental adhesives comprising
(a) 0.5 to 50% by weight of at least one calix[n]arene according to formula (I),
(b) 0.01 to 5% by weight of at least one initiator,
(c) 5 to 70% by weight of at least one additional monomer which can polymerize under radical conditions and/or at least one additional monomer which can polymerize by ring opening,
(d) 0 to 30% by weight of filler,
(e) 0.01 to 5% by weight of additives and
(f) 0 to 50% by weight of solvent,
the percentages each time adding up to 100%.

12. Use according to Claim 11, **characterized in that** the compositions comprise
(a) 1 to 30% by weight of a calix[n]arene according to formula (I),
(b) 0.01 to 2% by weight of at least one initiator,
(c) 5 to 60% by weight of at least one additional monomer which can polymerize under cationic and/or radical conditions and/or by ring opening,
(d) 3 to 20% by weight of filler,
(e) 0.01 to 3% by weight of additives and
(f) 0 to 20% by weight of solvent,
the percentages each time adding up to 100%.

## Revendications

1. Utilisation de compositions constituées par les composants
(a) 0,5 à 90% en poids d'au moins un calix[n]arène polymérisable selon la formule (I), 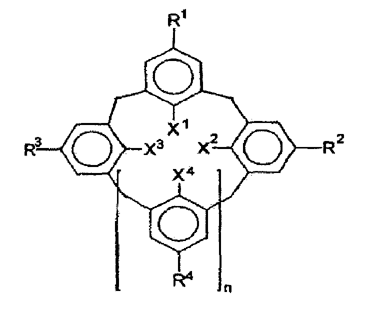 où
n = 1 à 5
R¹-R⁴= indépendamment l'un de l'autre H, un radical C₁- C₁₅-alkyle, qui peut être interrompu par O, un radical phényle ou un radical benzyle,
X¹ représente un groupe de structure : Y¹-Rₐ¹(Yₐ¹-R_{b}¹- PG¹)ₘ, où
Y¹ n'existe pas ou représente O, ester, amide, uréthane,
Rₐ¹ = un radical organique m-valent, qui peut contenir 1 à 30 atomes de carbone et le cas échéant également 0 à 6 hétéroatomes tels que O, S ou N,
m = 1 à 3,
Yₐ¹ n'existe pas ou représente O, ester, amide, uréthane,
R_{b}¹ n'existe pas ou représente un radical C₁-C₁₆- alkylène, qui peut être interrompu par des atomes d'oxygène,
PG¹ = un groupe polymérisable, un groupe polymérisable par voie radicalaire, tel que (méth)acrylate, (méth)acrylamide, vinyle, allyle ou styryle ; un groupe cyclique polymérisable par voie radicalaire avec ouverture de cycle, tel que par exemple les groupes 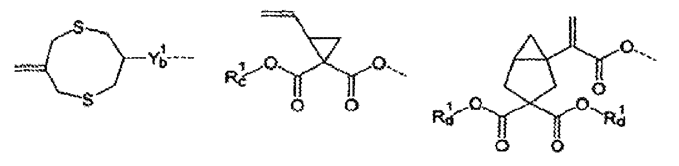 ou un groupe polymérisable par voie cationique, tel que par exemple un groupe vinyléther, glycidyle, époxyde ou oxétane cycloaliphatique ou un groupe nitro apte à une polyréaction, avec
Y_{b}¹ n'existe pas ou représente O, ester, amide, uréthane,
R_{c}¹, R_{d}¹ = indépendamment l'un de l'autre un radical C₁- C₁₅-alkyle qui peut être interrompu par O, un radical phényle, ou un radical benzyle ; et avec en outre X²-X⁴ indépendamment l'un de l'autre, n'existent pas ou représentent OH ou un radical C₁-C₁₀-alkyle et peuvent présenter indépendamment l'un de l'autre la même signification que X¹ et peuvent en outre représenter un groupe de structure : (Yₐ²-R_{b}²-HG)ₚ, où
Yₐ² n'existe pas ou représente O, ester, amide, uréthane,
R_{b}² = un radical organique p-valent, qui peut contenir 1 à 20 atomes de carbone et le cas échéant également 0 à 4 hétéroatomes tels que O ou N,
p = 1 à 3 et
HG = un groupe d'adhérence tel que -P=O(OH)₂ ; -O- P=O(OH)₂, -COOH ou -O-SO₂OH
(b) 0,01 à 5% en poids d'initiateur,
(c) 0 à 90% en poids d'au moins un autre monomère polymérisable par voie cationique et/ou radicalaire et/ou polymérisable avec ouverture de cycle,
(d) 0 à 85% en poids de charge et
(e) 0,01 à 5% en poids d'additifs
où les indications en pour cent s'ajoutent à chaque fois à 100%, pour la préparation de matériaux dentaires.

2. Utilisation selon la revendication 1, contenant
(a) 0,5 à 40% en poids d'au moins un calix[n]arène selon la formule (I),
(b) 0,01 à 2% en poids d'initiateur
(c) 1 à 70% en poids d'au moins un autre monomère polymérisable par voie cationique et/ou radicalaire et/ou polymérisable avec ouverture de cycle,
(d) 3 à 80% en poids de charge et
(e) 0,01 à 3% en poids d'additifs,
où les indications en pour cent s'ajoutent à chaque fois à 100%.

3. Utilisation selon la revendication 2, contenant des calix[n]arènes de formule (I) : 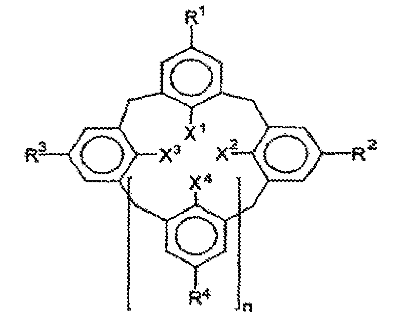 où
n = 1 à 3,
R¹-R⁴ = indépendamment l'un de l'autre H, un radical C₁- C₁₀-alkyle, qui peut être interrompu par O, ou un radical benzyle,
X¹ représente un groupe de structure : Y¹-Rₐ¹(Yₐ¹-R_{b}¹- PG¹)ₘ, dans lequel
Y¹ n'existe pas ou représente O ou ester,
Rₐ¹ = un radical organique m-valent, qui peut contenir 1 à 15 atomes de carbone et le cas échéant également 0 à 3 atomes d'oxygène,
m = 1 à 2,
Yₐ¹ n'existe pas ou représente O ou ester,
R_{b}¹ n'existe pas ou représente un radical C₁-C₁₆- alkylène, qui peut être interrompu par des atomes d'oxygène,
PG¹ = un groupe polymérisable, par exemple un groupe polymérisable par voie radicalaire, tel que (méth)acrylate, (méth)acrylamide, un groupe cyclique polymérisable par voie radicalaire avec ouverture de cycle, tel que par exemple les groupes 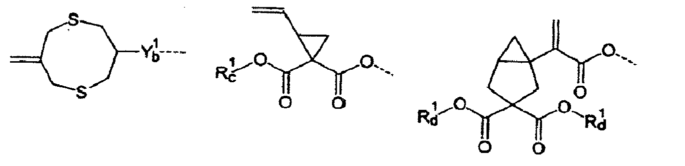 ou un groupe polymérisable par voie cationique, tel que par exemple un groupe époxyde ou oxétane cycloaliphatique ou un groupe nitro apte à une polyréaction, avec
Y_{b}¹ n'existe pas ou représente O, ester, uréthane,
R_{c}¹, R_{d}¹ = indépendamment l'un de l'autre, un radical C₁- C₅-alkyle, un radical phényle, ou un radical benzyle ;
et avec en outre
X²-X⁴ indépendamment l'un de l'autre, n'existent pas ou représentent un radical C₁-C₁₀-alkyle et peuvent présenter indépendamment l'un de l'autre la même signification que X¹ et peuvent en outre représenter un groupe de structure : (Yₐ²-R_{b}²-HG)ₚ, où
Yₐ² n'existe pas ou représente O ou ester,
R_{b}² = un radical organique p-valent, qui peut contenir 1 à 10 atomes de carbone et le cas échéant également 0 à 2 atomes d'oxygène,
p = 1 à 2 et
HG = un groupe d'adhérence tel que par exemple -P=O(OH)₂ ; -O-P=O(OH)₂, -COOH ou -O-SO₂OH.

4. Utilisation selon l'une quelconque des revendications précédentes, **caractérisée en ce que** sont contenus comme monomères polymérisables par voie radicalaire des monomères polymérisables avec ouverture de cycle, en particulier des vinylcyclopropanes monofonctionnels ou polyfonctionnels ou des cyclopropanacrylates bicycliques ou des allylsulfures cycliques et des (méth)acrylates polyfonctionnels ou des mélanges de ces monomères.

5. Utilisation selon l'une quelconque des revendications précédentes, **caractérisée en ce que** les compositions sont des ciments, contenant
(a) 0,5 à 30% en poids d'au moins un calix[n]arène selon la formule (I),
(b) 0,01 à 2% en poids d'initiateur
(c) 1 à 30% en poids d'au moins un autre monomère polymérisable par voie cationique et/ou radicalaire et/ou d'au moins un autre monomère polymérisable avec ouverture de cycle,
(d) 5 à 70% en poids de charge et
(e) 0,01 à 5% en poids d'additifs
où les indications en pour cent s'ajoutent à chaque fois à 100%.

6. Utilisation selon la revendication 5, **caractérisée en ce que** les compositions contiennent
(a) 0,5 à 20% en poids d'au moins un calix[n]arène selon la formule (I),
(b) 0,01 à 1,5% en poids d'initiateur,
(c) 5 à 20% en poids d'au moins un autre monomère polymérisable par voie radicalaire et/ou d'au moins un autre monomère polymérisable avec ouverture de cycle,
(d) 10 à 60% en poids de charge et
(e) 0,01 à 3% en poids d'additifs
où les indications en pour cent s'ajoutent à chaque fois à 100%.

7. Utilisation selon l'une quelconque des revendications 1 à 4, **caractérisée en ce que** les compositions sont des composites pour plombages, contenant
(a) 0,5 à 30% en poids d'au moins un calix[n]arène selon la formule (I),
(b) 0,01 à 5% en poids d'initiateur,
(c) 1 à 30% en poids d'au moins un autre monomère polymérisable par voie ionique ou radicalaire et/ou d'au moins un autre monomère polymérisable avec ouverture de cycle,
(d) 5 à 85% en poids de charge et
(e) 0,01 à 5% en poids d'additifs
où les indications en pour cent s'ajoutent à chaque fois à 100%.

8. Utilisation selon la revendication 7, **caractérisée en ce que** les compositions contiennent
(a) 0,5 à 20% en poids d'au moins un calix[n]arène selon la formule (I),
(b) 0,01 à 2% en poids d'initiateur
(c) 5 à 20% en poids d'au moins un autre monomère polymérisable par voie radicalaire et/ou d'au moins un autre monomère polymérisable avec ouverture de cycle,
(d) 10 à 80% en poids de charge et
(e) 0,01 à 3% en poids d'additifs
où les indications en pour cent s'ajoutent à chaque fois à 100%.

9. Utilisation selon l'une quelconque des revendications 1 à 4, **caractérisée en ce que** les compositions sont des matériaux de revêtement, contenant
(a) 0,5 à 70% en poids d'au moins un calix[n]arène selon la formule (I),
(b) 0,01 à 5% en poids d'initiateur,
(c) 5 à 60% en poids d'au moins un autre monomère polymérisable par voie ionique ou radicalaire et/ou d'au moins un autre monomère polymérisable avec ouverture de cycle,
(d) 1 à 30% en poids d'une charge
(e) 0,01 à 5% en poids d'additifs et
(f) 0 à 70% en poids de solvant,
où les indications en pour cent s'ajoutent à chaque fois à 100%.

10. Utilisation selon la revendication 9, **caractérisée en ce que** les compositions contiennent
(a) 1 à 50% en poids d'au moins un calix[n]arène selon la formule (I),
(b) 0,01 à 1,5% en poids d'initiateur,
(c) 5 à 60% en poids d'au moins un autre monomère polymérisable par voie radicalaire et/ou d'au moins un autre monomère polymérisable avec ouverture de cycle,
(d) 3 à 20% en poids d'une charge
(e) 0,01 à 3% en poids d'additifs et
(f) 0 à 30% en poids de solvant,
où les indications en pour cent s'ajoutent à chaque fois à 100%.

11. Utilisation selon l'une quelconque des revendications 1 à 4, **caractérisée en ce que** les compositions sont des adhésifs dentaires, contenant
(a) 0,5 à 50% en poids d'au moins un calix[n]arène selon la formule (I),
(b) 0,01 à 5% en poids d'au moins un initiateur
(c) 5 à 70% en poids d'au moins un autre monomère polymérisable par voie radicalaire et/ou d'au moins un autre monomère polymérisable avec ouverture de cycle,
(d) 0 à 30% en poids de charge
(e) 0,01 à 5% en poids d'additifs et
(f) 0 à 50% en poids de solvant
où les indications en pour cent s'ajoutent à chaque fois à 100%.

12. Utilisation selon la revendication 11, **caractérisée en ce que** les compositions contiennent
(a) 1 à 30% en poids d'un calix[n]arène selon la formule (I),
(b) 0,01 à 2% en poids d'au moins un initiateur
(c) 5 à 60% en poids d'au moins un autre monomère polymérisable par voie cationique et/ou radicalaire et/ou avec ouverture de cycle,
(d) 3 à 20% en poids de charge
(e) 0,01 à 3% en poids d'additifs et
(f) 0 à 20% en poids de solvant,
où les indications en pour cent s'ajoutent à chaque fois à 100%.
